# EUROPÄISCHE PATENTSCHRIFT

(11) **EP 3 558 409 B1**
(45) Veröffentlichungstag und Bekanntmachung des Hinweises auf die Patenterteilung: **16.11.2022**
(21) Anmeldenummer: 17825229.2
(22) Anmeldetag: 22.12.2017
(51) Int. Cl.: A61M 1/00

(54) **SAUGERSPITZE ZUM SCHONENDEN ABSAUGEN EINER BIOLOGISCHEN FLÜSSIGKEIT**
SUCTION TIP FOR THE GENTLE SUCTIONING OF A BIOLOGICAL FLUID
POINTE DE DISPOSITIF DE SUCCION CONÇUE POUR ASPIRER SOIGNEUSEMENT UN LIQUIDE BIOLOGIQUE

(30) Priorität: 23.12.2016 DE 102016125556
(43) Veröffentlichungstag der Anmeldung: 30.10.2019
(73) Patentinhaber: Georg-August-Universität Göttingen Stiftung Öffentlichen Rechts Universitätsmedizin, 37075 Göttingen (DE)
(72) Erfinder: FRIEDRICH, Martin, 37120 Bovenden (DE)
(74) Vertreter: REHBERG HÜPPE + PARTNER
(86) Internationale Anmeldenummer: PCT/EP2017/084321
(87) Internationale Veröffentlichungsnummer: WO 2018/115409

(56) Entgegenhaltungen:
- US-A- 3 785 380
- US-A- 3 963 028
- US-A- 5 163 926
- US-A1- 2005 004 520
- US-A1- 2009 247 936

## Beschreibung

### TECHNISCHES GEBIET DER ERFINDUNG

Die Erfindung bezieht sich auf eine Saugerspitze zum Absaugen einer biologischen Flüssigkeit. Insbesondere bezieht sich die Erfindung auf eine Saugerspitze mit einem eine Hauptachse und eine Wandung aufweisenden Hauptkörper, einem auf der Hauptachse an dem Hauptkörper ausgebildeten und einen freien Anschlussquerschnitt definierenden Anschluss für eine Absaugleitung, und mehreren seitlich in den Hauptkörper eintretenden, durch die Wandung des Hauptkörpers hindurch verlaufenden und in dem Hauptkörper mit dem Anschluss kommunizierenden dezentralen Sauglöchern.

Unter einer biologischen Flüssigkeit wird hier eine Flüssigkeit verstanden, die biologischer Herkunft ist oder die biologisches Material wie biologische Zellen, insbesondere lebende biologische Zellen, oder größere organische Moleküle, z. B. Antikörper, Oligopeptide, Proteine oder DNS, enthält, die für biologische Prozesse von Bedeutung sind. Bei der abzusaugenden biologischen Flüssigkeit kann es sich speziell um eine Körperflüssigkeit eines Tieres oder eines Menschen handeln. Die Körperflüssigkeit kann im Rahmen eines medizinischen Eingriffs abgesaugt werden. Noch spezieller ist die abzusaugende biologische Flüssigkeit Blut. Das Blut kann konkret aus einem Operationsfeld abgesaugt werden.

Wenn hier oder an anderen Stellen dieser Anmeldung von einem Tier die Rede ist, impliziert dies, dass es sich bei dem Tier nicht um einen Menschen handelt.

Beim Absaugen von Blut aus Operationsfeldern, um dieses an den Patienten zurückzugeben, an dem die jeweilige Operation durchgeführt wird, treten verschiedene Problematiken auf. Hierzu zählt das Vermischen des Bluts mit Umgebungsluft in Form von Luftblasen, das Schädigen von Bestandteilen des Bluts, inklusive und insbesondere der roten Blutkörperchen, weißen Blutkörperchen, Plaketten und Thrombozyten, durch Scherkräfte und das Festsaugen einer zum Absaugen verwendeten Saugerspitze an angrenzendem Gewebe des Patienten. Mit dem Blut vermischte Umgebungsluft muss wieder entfernt werden, bevor das Blut an den Patienten zurückgegeben werden darf, um Luftembolien zu verhindern. Wenn durch Scherkräfte geschädigtes Blut an einen Patienten zurückgegeben wird, kann dies zu nicht kalkulierbaren Schädigungen führen, u. a. Nierenversagen, Lungenschädigung, Thrombosen, Wundheilungsstörungen, systemische Inflammationsreaktionen. Das Festsaugen einer Saugerspitze an Gewebe ist mit der Gefahr der Schädigung dieses Gewebes verbunden. Zudem können bei einer sich intermittierend festsaugenden Saugerspitze sehr große Scherkräfte auf das abgesaugte Blut auftreten, während eine dauerhaft festgesaugte Saugerspitze funktionslos ist.

Entsprechende Problematiken treten beim Absaugen anderer biologischer Flüssigkeiten auf, wie u. a. unerwünschte Aktivierungen, Fehlaktivierungen, Strukturänderungen an Molekülen wie Faltungen, Denaturierungen, Zerfall und ähnliches.

### STAND DER TECHNIK

Aus der WO 2012/ 092 948 A1 ist eine Absaugvorrichtung zur Absaugung von Blut aus Operationsfeldern bekannt. Sie weist einen Sauger mit einer frontseitigen Saugöffnung an seinem distalen Ende sowie mehreren seitlich angeordneten Saugöffnungen und eine mit dem Sauger verbundene Pumpe auf. Die Pumpe erzeugt einen Saug-Unterdruck; und mit einer Steuereinrichtung wird eine an den Saugöffnungen wirksame Saugleistung eingestellt. Die Steuereinrichtung ist mit einem Schallwellensensor verbunden, der durch den Sauger bei dessen Betrieb erzeugte Schallwellen und andere Schwingungen aufnimmt. Wenn die Steuereinrichtung mittels des Schallwellensensors ein charakteristisches Schallwellenmuster detektiert, das für ein schlürfendes Sauggeräusch steht, reduziert sie die Saugleistung an den Saugöffnungen, weil das schlürfende Sauggeräusch auf ungünstige Absaugbedingungen hinweist, die mit der Gefahr u. a. einer Schädigung von Blutbestandteilen, einer Schädigung von Blutzellen oder anderer biologischer Agenzien verbunden sind. Zusätzlich ist die Steuereinrichtung mit einem Festsaug-Sensor verbunden, und beim Erkennen eines Festsaugens des Saugers mit dem Festsaug-Sensor reduziert sie ebenfalls die Saugleistung an den Saugöffnungen.

Aus der US 2014/ 0 276 486 A1 ist eine Absaugvorrichtung für Blut aus Operationsfeldern mit einer Saugspitze bekannt. Die Saugspitze weist einen hohlen Hauptkörper mit einer Mehrzahl von Öffnungen auf, die eine Fluidkommunikation zwischen der Umgebung und einem Innenraum des hohlen Hauptkörpers ermöglichen. Die Saugspitze weist weiterhin einen zylindrischen Fortsatz auf, der von dem hohlen Hauptkörper absteht und an den eine Absaugleitung angeschlossen ist. Der Hauptkörper ist kapselförmig mit abgerundeten Enden. Eine Öffnung des Hauptkörpers ist an seinem distalen Ende vorgesehen, die anderen Öffnungen an seinem Umfang.

Aus der US 7 955 318 B1 ist ein medizinisches Saugsystem mit Saugeinrichtungen zum Entfernen von Material aus Körperhöhlen während medizinischer Prozeduren bekannt. Das System ist an eine Vakuumquelle mit großem Anschlussquerschnitt, wie beispielsweise den Anschluss eines Vakuumsammelbehälters anschließbar. Das System umfasst eine Reihe von austauschbaren Saugerspitzen. Eine dieser Saugerspitzen weist eine atraumatische Form zum Reduzieren von Gewebetrauma während der medizinischen Prozedur auf. Die atraumatische Form umfasst eine im Wesentlichen abgerundete Oberfläche, und diese Saugerspitze wird auch als Saugkomponente mit stumpfer Spitze bezeichnet. Sie weist eine Mehrzahl von distalen Spitzeneinlässen und eine Mehrzahl von Seiteneinlässen auf, die zusammen ein feines siebartiges Saugfeld ausbilden.

Die WO 88/ 00 481 A1 beschreibt eine chirurgische Saugvorrichtung mit einer perforierten Saugerspitze zum Entfernen von chirurgischen Trümmern mit reduzierter Verstopfung und minimalem Trauma von angrenzendem Gewebe. Saugöffnungen sind derart an der Saugerspitze angeordnet, dass die Saugöffnungen, die unblockiert bleiben, wenn chirurgische Trümmer in anderen Saugöffnungen hängenbleiben, als Vakuummodulatoren wirken, die das Entfernen der Blockade erleichtern. Darüber hinaus ist die Wahrscheinlichkeit des Blockierens aller Saugöffnungen und damit des Ansaugens und Beschädigens von Gewebe reduziert. Konkret sind sowohl an einer Vorderseite als auch einer Rückseite der knollenförmigen Saugerspitze Sauglöcher vorgesehen, die zu einem Zentrum der knollenförmigen Saugerspitze hin verlaufen.

Aus der US 5 827 218 A ist eine Saugerspitze für eine chirurgische Spülvorrichtung bekannt. Die Saugerspitze umfasst ein äußeres Rohr mit einem distalen Endbereich zur Kommunikation mit einem Operationsort und ein Innenrohr, das sich in dem Außenrohr erstreckt und ein offenes distales Ende zur Kommunikation mit dem Operationsort aufweist. Die Saugerspitze ist ausgebildet, um während des Absaugens Turbulenz nahe empfindlicher Organe zu minimieren. Ein Turbulenz minimierender distaler Endbereich des äußeren Rohrs weist ein konvex abgerundetes Ende mit in Umfangsrichtung und axial beabstandeten Saugströmungslöchern durch die Wand des äußeren Rohrs in sein hohles Inneres auf. Die Saugströmungslöcher sind in sich axial erstreckenden, in Umfangsrichtung beabstandeten Reihen angeordnet, wobei jede Reihe ein nach vorne zeigendes Loch am gerundeten Ende und sechs sich radial erstreckende Löcher umfasst.

Aus der US 3 963 028 A ist ein Saugstab zur Verwendung bei chirurgischen Eingriffen bekannt. Eine Saugerspitze ist auf das proximale Ende einer rohrförmigen Sonde des Saugstabs aufgestzt. Die Saugerspitze weist Sauglöcher in direkter Fluidkommunikation mit einem zentralen Durchgangsloch in der rohrförmigen Sonde auf. Die Saugerspitze mit den Sauglöchern ist als Sieb zum Verhindern des Einsaugens von Gewebematerial vorgesehen. Die Sauglöcher umfassen ein koaxial zu dem Durchgangsloch der rohrförmigen Sonde verlaufendes zentrales Saugloch mit größerem Durchmesser und parallel zu dem zentralen Saugloch verlaufende dezentrale Sauglöcher. Alle Sauglöcher münden geradlinig in das Durchgangsloch der Sonde ein.

Aus der US 5 163 926 A ist eine Saugvorrichtung bekannt, bei der eine Saugerspitze an den distalen Enden eines Saugrohrs und eines parallel zu Saugrohr verlaufenden Versorgungsrohrs für ein Antikoagulationsmittel angeordnet ist. In der Saugerspitze wird durch Löcher in der Saugerspitze eingesaugtes Blut mit dem Antikoagulationsmittel aus dem Versorgungsrohr vermischt und dann über das Saugrohr abgesaugt. Die Saugerspitze ist halbkugelkalottenförmig, und ein zentrales Loch sowie mehrere dezentrale Sauglöcher in der Saugerspitze erstrecken sich jeweils radial zu einem Kugelmittelpunkt der Halbkugelkalotte. Ein zentrales Sauglochoch der Saugerspitze verläuft dabei zwar parallel aber soweit exzentrisch zu dem angeschlossenen Saugrohr, dass es dessen Anschlussquerschnitt nicht trifft.

Aus der US 2005/ 0 004 520 A1 ist eine Absaugvorrichtung mit einem festen Körper bekannt, an den eine Saugerspitze ansetzbar ist. Ein von der Saugerspitze kommendes Hauptfluidstrom tritt in einen Haupteinlass mit konstantem Querschnitt bis zum Auslass der Absaugvorrichtung ein. Wenn der Haupteinlass verstopft ist, wird Luft durch dünne langgestreckte Bypassöffnungen in den festen Körper eingesaugt und vereinigt sich dort mit dem Hauptfluidstrom unter flachen Auftreffwinkeln. Dadurch folgen die Fluidströme glatten Pfaden und vereinigen sich problemlos zu einer kombinierten Fluidströmung am Auslass der Absaugvorrichtung.

Aus der US 3 785 380 A ist eine Blutabsaugvorrichtung bekannt, deren am proximalen Ende eines Saugrohrs ausgebildete Saugerspitze neben einem durch das offene Ende des Saugrohrs gebildeten zentralen axialen Absaugloch mehrere dezentrale Absauglöcher kleineren Durchmessers aufweist, die radial durch die Saugleitung verlaufen. In einem Gehäuse der Blutabsaugvorrichtung, an das die Saugerspitze über das Saugrohr angeschlossen ist, wird das abgesaugte Blut durch einen Filter geführt.

Aus der US 2009/ 0 247 936 A1 ist ein chirurgisches Instrument mit einer Ultraschallschneidspitze bekannt, das die Merkmale des Oberbegriffs des Patentanspruchs 1 aufweist. In einer Basis der Ultraschallschneidspitze ist ein Paar von Absauglöchern vorgesehen. Die Absauglöcher verlaufen beiderseits einer Ultraschallschneidklinge unter einem Schrägwinkel zu einem zentralen Absaugkanal und gehen gemeinsam in diesen Absaugkanal über.

### AUFGABE DER ERFINDUNG

Der Erfindung liegt die Aufgabe zugrunde, eine Saugerspitze zum Absaugen einer biologischen Flüssigkeit aufzuzeigen, deren geometrische Ausgestaltung die eingangs für das Beispiel des Absaugens von Blut aus Operationsfeldern geschilderten Problematiken minimiert.

### LÖSUNG

Die Aufgabe der Erfindung wird durch eine Saugerspitze mit den Merkmalen des unabhängigen Patentanspruchs 1 gelöst. Die abhängigen Patentansprüche sind auf bevorzugte Ausführungsformen der erfindungsgemäßen Saugerspitze gerichtet.

### BESCHREIBUNG DER ERFINDUNG

Bei einer erfindungsgemäßen Saugerspitze zum Absaugen einer biologischen Flüssigkeit mit einem eine Hauptachse und eine Wandung aufweisenden Hauptkörper, einem auf der Hauptachse an dem Hauptkörper ausgebildeten und einen freien Anschlussquerschnitt definierenden Anschluss für eine Absaugleitung und mehreren seitlich in den Hauptkörper eintretenden, durch die Wandung des Hauptkörpers hindurch verlaufenden und in dem Hauptkörper mit dem Anschluss kommunizierenden dezentralen Sauglöchern überlappt ein freier Mindestquerschnitt jedes dezentralen Sauglochs bei kontinuierlicher Fortsetzung dessen Verlaufs durch die Wandung des Hauptkörpers vollständig mit dem freien Anschlussquerschnitt der Saugerspitze.

Wie schon oben angemerkt wurde, ist unter einer biologischen Flüssigkeit, für deren Absaugung die erfindungsgemäße Saugerspitze vorgesehen ist, jede Flüssigkeit zu verstehen, die biologischer Herkunft ist oder biologisches Material wie biologische Zellen, insbesondere lebende biologische Zellen, oder größere organische Moleküle, z. B. Antikörper, Oligopeptide, Proteine oder DNS, enthält, die für biologische Prozesse von Bedeutung sind.

Die mehreren dezentralen Sauglöcher durch die Wandung des Hauptkörpers der erfindungsgemäßen Saugerspitze sind insoweit dezentral, als dass sie seitlich, d. h. unter einem Winkel eindeutig größer null zu der Hauptachse, in den Hauptkörper eintreten, so dass sie dem zentral auf der Hauptachse ausgebildeten Anschluss für die Absaugleitung nicht direkt gegenüberliegen.

Unter der kontinuierlichen Fortsetzung des Verlaufs des jeweiligen dezentralen Sauglochs durch die Wandung des Hauptkörpers ist die Extrapolation des Verlaufs des jeweiligen dezentralen Sauglochs durch die Wandung durch einen etwaigen Hohlraum in dem Hauptkörper hindurch bis in den Bereich der Hauptachse des Hauptkörpers zu verstehen. Bei einem geraden Verlauf des dezentralen Sauglochs durch die Wandung ist dessen kontinuierliche Fortsetzung auch gerade. Bei einem gekrümmten Verlauf mit konstantem Krümmungsradius behält die kontinuierliche Fortsetzung den Krümmungsradius bei. Wenn der Verlauf des jeweiligen dezentralen Sauglochs durch die Wandung eine sich ändernde Krümmung aufweist, behält seine kontinuierliche Fortsetzung die Änderungsrate der sich ändernden Krümmung bei.

Dass der freie Mindestquerschnitt jedes dezentralen Sauglochs bei der kontinuierlichen Fortsetzung seines Verlaufs durch die Wandung des Hauptkörpers vollständig mit dem von dem Anschluss definierten freien Anschlussquerschnitt der Saugerspitze überlappt, impliziert, dass die kontinuierliche Fortsetzung des Verlaufs jedes der dezentralen Sauglöcher in den Anschluss der erfindungsgemäßen Saugerspitze hinein führt.

Mit dem von dem Anschluss definierten freien Anschlussquerschnitt der Saugerspitze ist der freie Querschnitt des Anschlusses selbst oder der daran anzuschließenden Absaugleitung gemeint, je nachdem, welcher Querschnitt kleiner ist und damit im Betrieb den freien Anschlussquerschnitt der Saugerspitze bestimmt. Auch wenn dies der freie Querschnitt der Absaugleitung ist, wird er durch den für die Absaugleitung vorgesehenen Anschluss definiert.

Mit dem freien Mindestquerschnitt jedes dezentralen Sauglochs ist dessen kleinster freier Querschnitt bei dessen Verlauf und senkrecht zu dessen Verlauf durch die Wandung des Hauptkörpers gemeint. Wenn dieser freie Mindestquerschnitt längs der kontinuierlichen Fortsetzung des Verlaufs des jeweiligen dezentralen Sauglochs durch die Wandung des Hauptkörpers auf den Anschluss projiziert wird, überlappt er vollständig mit dem freien Anschlussquerschnitt der Saugerspitze. Das heißt, die durch den freien Mindestquerschnitt jedes dezentralen Sauglochs eintretende biologische Flüssigkeit gelangt längs dieses Verlaufs zu dem und durch den Anschlussquerschnitt der Saugerspitze, ohne auf Strömungshindernisse zu stoßen. Dies sind optimale Voraussetzungen für eine laminare Strömung der biologischen Flüssigkeit durch die Saugerspitze und für eine Minimierung der auf die Bestandteile der durch die Saugerspitze strömenden biologischen Flüssigkeit einwirkenden Scherkräfte, was einen Schutz für die Integrität der biologischen Flüssigkeit bedeutet. Damit wird auch die Gefahr eines Vermischens der durch die Saugerspitze hindurch eingesaugten biologischen Flüssigkeit mit Umgebungsluft in Form von Luftblasen minimiert. Zudem wird eine reduzierte Geräuschentwicklung beim Absaugen der biologischen Flüssigkeit festgestellt, die sowohl als solche vorteilhaft als auch ein Indiz für Absaugbedingungen ist, die die biologische Flüssigkeit schonen.

Eine Summe von Flächen der freien Mindestquerschnitte der dezentralen Sauglöcher ist mindestens so groß wie eine Fläche des freien Anschlussquerschnitts der Saugerspitze. Dies bedeutet, dass die in die Saugerspitze eingesaugte biologische Flüssigkeit in der Saugerspitze weiter beschleunigt wird, wobei der Unterdruck, der zum Absaugen der biologischen Flüssigkeit aufgebracht wird, sukzessive und damit nicht schlagartig beim Eintreten der biologischen Flüssigkeit in die Sauglöcher abfällt. Der Abfall des Unterdrucks und die damit einhergehende Beschleunigung der biologischen Flüssigkeit in dem Hauptkörper der Saugerspitze sind bei der erfindungsgemäßen Saugerspitze unkritisch, weil die Strömung der biologischen Flüssigkeit in deren Hauptkörper auf keine Hindernisse trifft. Weiterhin verhindert die vergleichsweise große Summe der Flächen der freien Mindestquerschnitte der dezentralen Sauglöcher eine Neigung der erfindungsgemäßen Saugerspitze zum Festsaugen an Gewebe, das an eine Lache der biologischen Flüssigkeit angrenzt.

Konkret können die dezentralen Sauglöcher auf mindestens einem gedachten Ring um die Hauptachse in den Hauptkörper eintreten. Dabei kann eine Summe von Flächen der freien Mindestquerschnitte der dezentralen Sauglöcher auf jedem der gedachten Ringe um die Hauptachse mindestens so groß sein wie die Fläche des freien Anschlussquerschnitts der Saugerspitze. Die dezentralen Sauglöcher können auch auf mehreren gedachten Ringen um die Hauptachse in den Hauptkörper eintreten.

Für eine möglichst ruhige Strömung der durch die Sauglöcher angesaugten biologischen Flüssigkeit durch den Hauptkörper ist es von Vorteil, wenn die freien Mindestquerschnitte der dezentralen Sauglöcher bei der kontinuierlichen Fortsetzung ihrer Verläufe durch die Wandung des Hauptkörpers gleichmäßig über den freien Anschlussquerschnitt der Saugerspitze verteilt sind. So stören sich die Teilströmungen der biologischen Flüssigkeit durch die einzelnen dezentralen Sauglöcher so wenig wie möglich.

Beispielsweise sind die freien Mindestquerschnitte der dezentralen Sauglöcher kreisförmig.

Die dezentralen Sauglöcher treten unter einem spitzen Winkel von nicht mehr als 60° oder von nicht mehr als 45°, aber auch von nicht weniger als typischerweise 15° zu der Hauptachse in den Hauptkörper eintreten. Dabei können zumindest mehrere der dezentralen Sauglöcher einen geraden Verlauf durch die Wandung des Hauptkörpers aufweisen, wobei ihre freien Mindestquerschnitte aufgrund des spitzen Winkels trotzdem bei kontinuierlicher Fortsetzung dieses geraden Verlaufs vollständig mit dem freien Anschlussquerschnitt der Saugerspitze überlappen.

Zumindest mehrere der dezentralen Sauglöcher können aber auch einen gekrümmten Verlauf durch die Wandung des Hauptkörpers aufweisen. Dabei ist ein Krümmungsradius des gekrümmten Verlaufs bezogen auf eine Lochachse des jeweiligen Lochs vorzugsweise mindestens so groß wie ein maximaler Durchmesser des Hauptkörpers senkrecht zu seiner Hauptachse. Das heißt, der Krümmungsradius der dezentralen Sauglöcher mit gekrümmtem Verlauf ist vergleichsweise groß. Dies ist gleichbedeutend damit, dass die durch die Sauglöcher strömende biologische Flüssigkeit in den Sauglöchern keine starke Umlenkung erfährt, in deren Folge größere Scherkräfte auf die Bestandteile der biologischen Flüssigkeit auftreten könnten.

Neben den dezentralen Sauglöchern weist der Hauptkörper ein zentrales Saugloch auf. Dabei kann ein freier Mindestquerschnitt des zusätzlichen zentralen Sauglochs etwa genauso groß wie der freie Anschlussquerschnitt der Saugerspitze sein, hiervon aber auch um typischerweise maximal 20 Prozent nach oben oder unten abweichen.

Aus dem Grund eines möglichst gleichmäßigen Druckabfalls und einer entsprechend möglichst gleichmäßigen Beschleunigung der Strömung der biologischen Flüssigkeit durch den Hauptkörper kann jedes Saugloch eine zu dem Anschluss hin stetig abnehmende Fläche seines freien Querschnitts aufweisen. Alternativ oder zusätzlich kann ein freier Gesamtquerschnitt des Hauptkörpers über alle Sauglöcher und auch einen etwaigen Hohlraum in dem Hauptkörper hinweg betrachtet stetig, d. h. sprung- und vorzugsweise auch knickfrei in den freien Anschlussquerschnitt der Saugerspitze übergehen. Dies impliziert auch, dass alle Sauglöcher an ihren Enden abgerundete oder zumindest angefaste Kanten aufweisen.

In einer Ausführungsform der erfindungsgemäßen Saugerspitze ist in einem Hohlraum innerhalb des Hauptkörpers ein offenporiger Schwamm angeordnet, wobei die Sauglöcher durch den offenporigen Schwamm hindurch mit dem Anschluss kommunizieren. Dieser offenporige Schwamm kann in grundsätzlich bekannter Weise zum Entschäumen der in die Saugerspitze eingesaugten biologischen Flüssigkeit dienen. Er ist aber ausdrücklich nur eine Option bei der Saugerspitze, die auch einen ungefüllten Hohlraum innerhalb des Hauptkörpers oder gar keinen gegenüber den Sauglöchern abgrenzbaren Hohlraum innerhalb des Hauptkörpers aufweisen kann.

Bei bevorzugten Verwendungen der erfindungsgemäßen Saugerspitze ist die abzusaugende biologische Flüssigkeit eine Körperflüssigkeit eines Tieres oder eines Menschen. Die Körperflüssigkeit kann im Rahmen eines medizinischen Eingriffs abgesaugt werden. Damit ist aber diese Verwendung der erfindungsgemäßen Saugerspitze kein Verfahren zur chirurgischen oder therapeutischen Behandlung des menschlichen oder tierischen Körpers, der vom Patentschutz ausgenommen wäre, weil durch das Absaugen des Bluts der Körper des jeweiligen Patienten weder chirurgisch noch therapeutisch behandelt wird. Das gilt auch dann, wenn die abzusaugende biologische Flüssigkeit Blut ist, das aus einem Operationsfeld abgesaugt und/oder derart aufbereitet wird, dass es an einen Patienten, an dem eine Operation durchgeführt wird, zurückgegeben werden kann, weil die Operation an dem Patienten nicht Gegenstand der Verwendung der Saugerspitze ist.

Vorteilhafte Weiterbildungen der Erfindung ergeben sich aus den Patentansprüchen, der Beschreibung und den Zeichnungen. Die in der Beschreibung genannten Vorteile von Merkmalen und von Kombinationen mehrerer Merkmale sind lediglich beispielhaft und können alternativ oder kumulativ zur Wirkung kommen, ohne dass die Vorteile zwingend von erfindungsgemäßen Ausführungsformen erzielt werden müssen. Ohne dass hierdurch der Gegenstand der beigefügten Patentansprüche verändert wird, gilt hinsichtlich des Offenbarungsgehalts der ursprünglichen Anmeldungsunterlagen und des Patents Folgendes: weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die in separaten Zeichnungen dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen.

Die in den Patentansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von einer Absaugleitung die Rede ist, ist dies so zu verstehen, dass genau eine Absaugleitung, zwei Absaugleitungen oder mehr Absaugleitungen vorhanden sind. Die in den Patentansprüchen angeführten Merkmale können durch andere Merkmale ergänzt werden oder die einzigen Merkmale sein, die das jeweilige Erzeugnis aufweist.

Die in den Patentansprüchen enthaltenen Bezugszeichen stellen keine Beschränkung des Umfangs der durch die Patentansprüche geschützten Gegenstände dar. Sie dienen lediglich dem Zweck, die Patentansprüche leichter verständlich zu machen.

### KURZBESCHREIBUNG DER FIGUREN

Im Folgenden wird die Erfindung anhand in den Figuren dargestellter bevorzugter Ausführungsbeispiele weiter erläutert und beschrieben.
- **Fig. 1**: ist ein Längsschnitt durch eine erste Ausführungsform der erfindungsgemäßen Saugerspitze.
- **Fig. 2**: ist eine Seitenansicht der Saugerspitze gemäß Fig. 1, aber mit gegenüber Fig. 1 um 45° verdrehter Saugerspitze.
- **Fig. 3**: ist ein Längsschnitt durch eine zweite Ausführungsform der erfindungsgemäßen Saugerspitze.
- **Fig. 4**: ist ein Längsschnitt durch eine weitere Ausführungsform der erfindungsgemäßen Saugerspitze; und
- **Fig. 5**: illustriert die Überlappung von freien Mindestquerschnitten von dezentralen Sauglöchern mit einem freien Anschlussquerschnitt der Saugerspitze.

### FIGURENBESCHREIBUNG

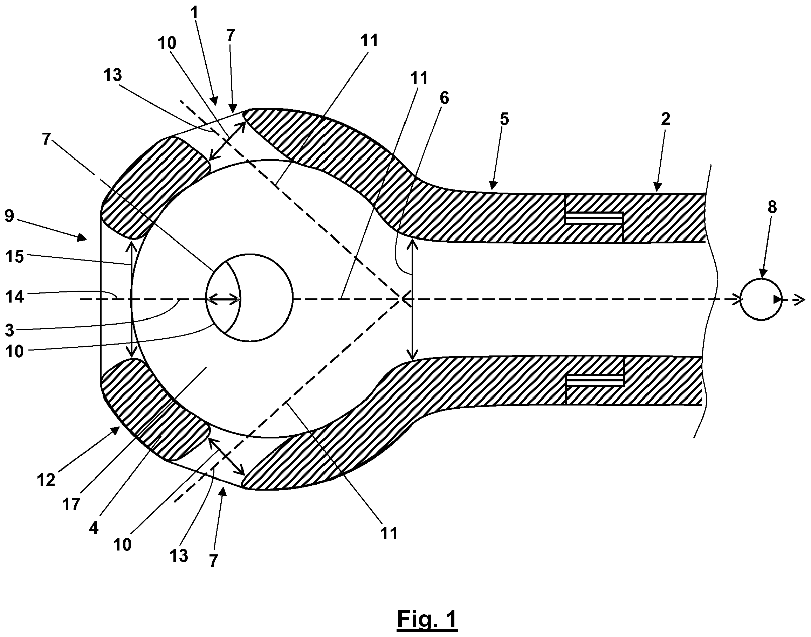

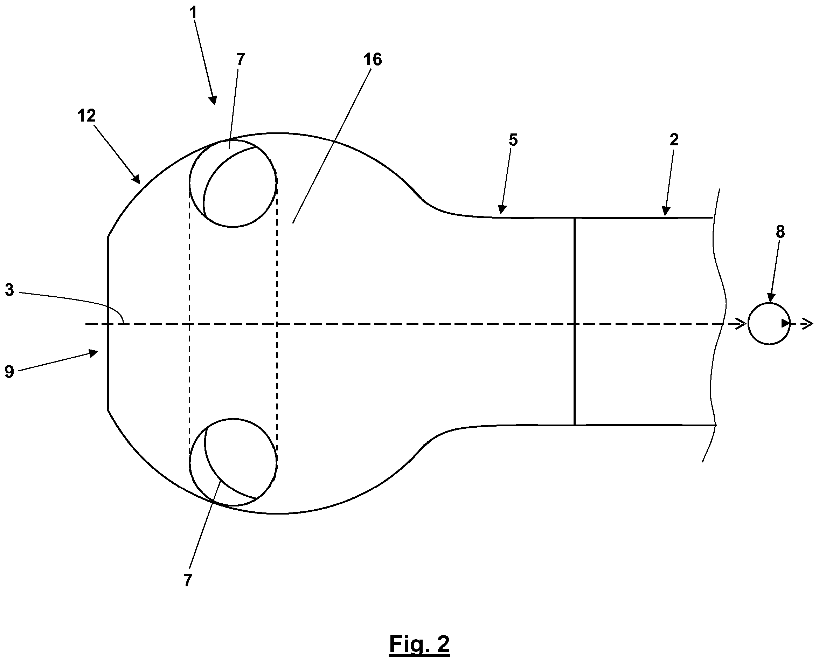

Die in den **Fig. 1** **und** **2** dargestellte Saugerspitze 1 dient zum Absaugen einer biologischen Flüssigkeit, hier konkret von Blut aus Operationsfeldern. Die Saugerspitze 1 ist über eine Absaugleitung 2 an eine hier nur symbolisch dargestellte Pumpeinrichtung 8 angeschlossen, mit der ein Unterdruck an die Saugerspitze 1 anlegbar ist. Dieser Unterdruck kann optional abhängig von hier nicht dargestellten Sensoren an der Saugerspitze 1 gesteuert werden. Die Saugerspitze 1 ist speziell dafür ausgestaltet, dass die abgesaugte biologische Flüssigkeit möglichst schonend bis in die Absaugleitung 2 hinein gelangt. Dafür sind insbesondere folgende Maßnahmen getroffen: Die Saugerspitze 1 weist einen Hauptkörper 12 mit einer Hauptachse 3 und einer Wandung 4 auf. An dem Hauptkörper 12 ist ein Anschluss 5 für die Absaugleitung 2 ausgebildet. Der Anschluss 5 definiert einen Anschlussquerschnitt 6, der gleich dem minimalen Querschnitt des Anschlusses 5 selbst und der daran angeschlossenen Absaugleitung 2 ist, die hier stufenlos und ohne Querschnittsveränderung ineinander übergehen. In den Hauptkörper treten mehrere dezentrale Sauglöcher 7 und ein zentrales Saugloch 9 ein. Die Sauglöcher 7 und 9 verlaufen durch die Wandung 4 des Hauptkörpers 12 und kommunizieren in dem Hauptkörper 12 mit dem Anschluss 5. Ein freier Mindestquerschnitt 10 jedes der dezentralen Sauglöcher 7 überlappt vollständig mit dem freien Anschlussquerschnitt 6 der Saugerspitze 1, wenn man den freien Mindestquerschnitt 10 längs einer kontinuierlichen Fortsetzung 11 des Verlaufs 13 des jeweiligen dezentralen Sauglochs 7 durch die Wandung 4 auf den Anschluss 5 projiziert. Der freie Mindestquerschnitt 10 ist dabei der kleinste freie Querschnitt des jeweiligen dezentralen Sauglochs 7 senkrecht zu dessen Verlauf 13 durch die Wandung 4. Das zentrale Saugloch 9 weist einen Verlauf 14 durch die Wandung 4 längs der Hauptachse 3 auf, und sein freier Mindestquerschnitt 15 ist deckungsgleich mit dem Anschlussquerschnitt 6. Die Summe der Flächen der freien Mindestquerschnitte 10 der dezentralen Sauglöcher 7 ist vorzugsweise mindestens genauso groß wie die Fläche des Anschlussquerschnitts 6. Der Verlauf 13 der dezentralen Sauglöcher 7 durch die Wandung 4 des Hauptkörpers 12 ist hier gerade. Entsprechend sind auch die kontinuierlichen Fortsetzungen 11 dieses Verlaufs gerade. Die dezentralen Sauglöcher 7 sind auf einem gedachten Ring 16 um die Hauptachse 3, d. h. rotationssymmetrisch zu der Hauptachse 3 angeordnet, wie insbesondere Fig. 2 zeigt.

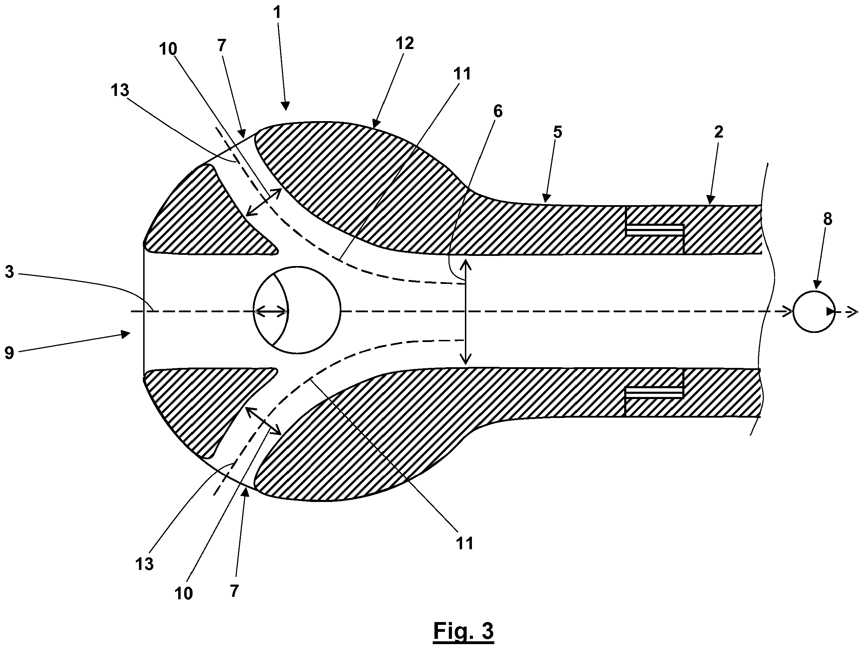

Während der Hauptkörper 12 der Saugerspitze 1 gemäß den Fig. 1 und 2 einen Hohlraum 17 umschließt, durch den hindurch die Sauglöcher 7 und 9 mit dem Anschluss 5 kommunizieren, ist ein solcher gegenüber den Sauglöchern 7 und 9 abgrenzbarer Hohlraum in dem Hauptkörper 12 der Saugerspitze 1 gemäß **Fig. 3** nicht vorhanden. Hier vereinigen sich die freien Querschnitte der Sauglöcher 7 und 9 bei ihrem Übergang in den Anschluss 5. Zudem sind die Verläufe 13 der dezentralen Absauglöcher 7 durch die Wandung 4 des Hauptkörpers 12 nicht geradlinig, sondern gekrümmt. Entsprechend sind auch ihre kontinuierlichen Fortsetzungen zu dem Anschluss 5 hin gekrümmt. Aber auch hier gilt, dass bei Projektion der freien Mindestquerschnitte 10 der dezentralen Absauglöcher 7 längs der kontinuierlichen Fortsetzungen 11 der Verläufe 13 durch die Wandung 4 eine vollständige Überlappung der freien Mindestquerschnitte 10 mit dem Anschlussquerschnitt 6 gegeben ist.

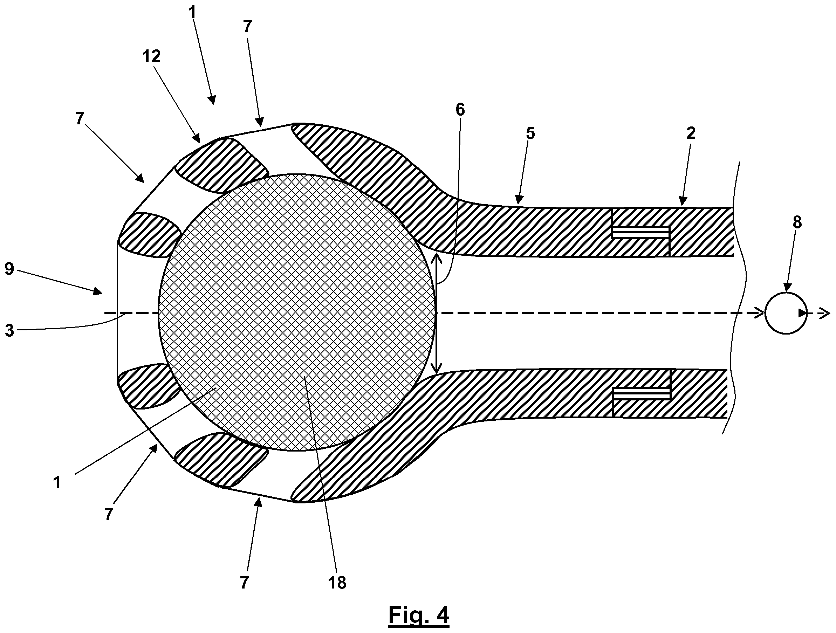

Bei der Saugerspitze 1 gemäß **Fig. 4** weist der Hauptkörper 12 wieder einen Hohlraum 17 auf. Dieser ist hier jedoch von einem offenporigen Schwamm 18 ausgefüllt, durch den hindurch die Sauglöcher 7 und 9 mit dem Anschluss 5 in dem Hauptkörper 12 kommunizieren. Zudem sind die dezentralen Sauglöcher 7 hier auf zwei in Richtung der Hauptachse 3 voneinander beabstandeten gedachten Ringen angeordnet. Dabei ist die Summe der Flächen der freien Mindestquerschnitte der Absauglöcher 7 auf jedem Ring vorzugsweise mindestens genauso groß wie die Fläche des Anschlussquerschnitts 6.

Alle Längsschnitte in den Fig. 1, 3 und 4 zeigen, dass alle Sauglöcher 7 und 9 sowohl im Bereich ihres Eintritts in den Hauptkörper 12 als auch im Bereich ihres Wiederaustritts aus der Wandung 4 des Hauptkörpers 12 abgerundete Kanten aufweisen.

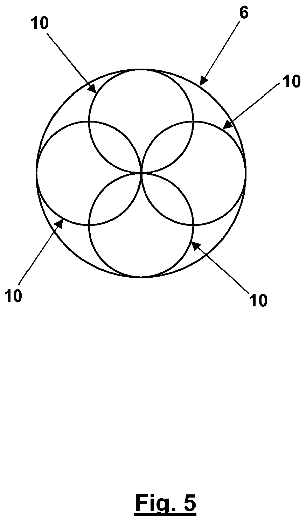

**Fig. 5** illustriert ein Beispiel für die Überlappung der auf den freien Anschlussquerschnitt 6 längs der kontinuierlichen Fortsetzungen 11 der Verläufe 13 projizierten freien Mindestquerschnitte 10 der dezentralen Sauglöcher 7 der erfindungsgemäßen Saugerspitze 1. Es ist eine vollständige Überlappung jedes dieser freien Mindestquerschnitte 10 mit dem Anschlussquerschnitt 6 gegeben. Wenn, wie hier angedeutet, vier Mindestquerschnitte 10 jeweils den halben Durchmesser wie der Anschlussquerschnitt 6 haben, ist die Summe der Flächen der freien Mindestquerschnitte 10 genauso groß wie die Fläche des freien Anschlussquerschnitts 6.

### BEZUGSZEICHENLISTE

- 1: Saugerspitze
- 2: Absaugleitung
- 3: Hauptachse
- 4: Wandung
- 5: Anschluss
- 6: freier Anschlussquerschnitt
- 7: dezentrales Saugloch
- 8: Pumpeinrichtung
- 9: zentrales Saugloch
- 10: freier Mindestquerschnitt des dezentralen Sauglochs 7
- 11: kontinuierliche Fortsetzung des Verlaufs 13
- 12: Hauptkörper
- 13: Verlauf des dezentralen Sauglochs 7 durch die Wandung 4
- 14: Verlauf des zentralen Sauglochs 9 durch die Wandung 4
- 15: freier Mindestquerschnitt des zentralen Sauglochs 9 durch die Wandung 4
- 16: gedachter Ring
- 17: Hohlraum
- 18: offenporiger Schwamm

## Patentansprüche

1. Saugerspitze (1) zum Absaugen einer biologischen Flüssigkeit mit
- einem eine Hauptachse (3) und eine Wandung (4) aufweisenden Hauptkörper (12),
- einem auf der Hauptachse (3) an dem Hauptkörper (12) ausgebildeten und einen freien Anschlussquerschnitt (6) der Saugerspitze (1) definierenden Anschluss (5) für eine Absaugleitung (2) und
- mehreren seitlich in den Hauptkörper (12) eintretenden, durch die Wandung (4) des Hauptkörpers (12) hindurch verlaufenden und in dem Hauptkörper (12) mit dem Anschluss (5) kommunizierenden dezentralen Sauglöchern (7),
- wobei die dezentralen Sauglöcher (7) unter einem spitzen Winkel von nicht mehr als 60° zu der Hauptachse (3) derart seitlich in den Hauptkörper (12) eintreten, dass sie dem zentral auf der Hauptachse (3) ausgebildeten Anschluss (5) für die Absaugleitung (2) nicht direkt gegenüberliegen,
- wobei ein freier Mindestquerschnitt (10) jedes dezentralen Sauglochs (7) bei kontinuierlicher Fortsetzung dessen Verlaufs (13) durch die Wandung (4) des Hauptkörpers (12) vollständig mit dem freien Anschlussquerschnitt (6) der Saugerspitze (1) überlappt und
- wobei eine Summe von Flächen der freien Mindestquerschnitte (10) der dezentralen Sauglöcher (7) mindestens so groß ist wie eine Fläche des freien Anschlussquerschnitts (6) der Saugerspitze (1),
**dadurch gekennzeichnet,**
- **dass** der Hauptkörper (12) weiterhin ein zentrales Saugloch (9) aufweist, das längs der Hauptachse (3) durch die Wandung (4) verläuft.

2. Saugerspitze (1) nach Anspruch 1, **dadurch gekennzeichnet, dass** ein freier Mindestquerschnitt (15) des zentralen Sauglochs (9) deckungsgleich mit dem freien Anschlussquerschnitt (6) der Saugerspitze (1) ist.

3. Saugerspitze (1) nach einem der vorhergehenden Ansprüche, **dadurch gekennzeichnet, dass** die dezentralen Sauglöcher (7) auf mindestens einem gedachten Ring (16) um die Hauptachse (3) in den Hauptkörper (12) eintreten.

4. Saugerspitze (1) nach Anspruch 3, **dadurch gekennzeichnet, dass** eine Summe von Flächen der freien Mindestquerschnitte (10) der dezentralen Sauglöcher (7) auf jedem der gedachten Ringe (16) um die Hauptachse (3) mindestens so groß ist wie eine Fläche des freien Anschlussquerschnitts (6) der Saugerspitze (1).

5. Saugerspitze (1) nach einem der vorhergehenden Ansprüche, **dadurch gekennzeichnet, dass** die freien Mindestquerschnitte (10) der dezentralen Sauglöcher (7) bei der kontinuierlichen Fortsetzung ihrer Verläufe (13) durch die Wandung (4) des Hauptkörpers (12) gleichmäßig über den freien Anschlussquerschnitt (6) der Saugerspitze (1) verteilt sind.

6. Saugerspitze (1) nach einem der vorhergehenden Ansprüche, **dadurch gekennzeichnet, dass** die freien Mindestquerschnitte (6) der dezentralen Sauglöcher (7) kreisförmig sind.

7. Saugerspitze (1) nach einem der vorhergehenden Ansprüche, **dadurch gekennzeichnet, dass** die dezentralen Sauglöcher (7) unter einem spitzen Winkel von nicht mehr als 45° zu der Hauptachse (3) in den Hauptkörper (12) eintreten.

8. Saugerspitze (1) nach Anspruch 7, **dadurch gekennzeichnet, dass** zumindest mehrere der dezentralen Sauglöcher (7) einen geraden Verlauf (13) durch die Wandung (4) des Hauptkörpers (12) aufweisen.

9. Saugerspitze (1) nach einem der vorhergehenden Ansprüche, **dadurch gekennzeichnet, dass** zumindest mehrere der dezentralen Sauglöcher (7) einen gekrümmten Verlauf (13) durch die Wandung (4) des Hauptkörpers (12) aufweisen.

10. Saugerspitze (1) nach Anspruch 9, **dadurch gekennzeichnet, dass** ein Krümmungsradius des gekrümmten Verlaufs bezogen auf eine Lochachse des jeweiligen Lochs mindestens so groß ist wie ein maximaler Durchmesser des Hauptkörpers (12) senkrecht zu seiner Hauptachse (3).

11. Saugerspitze (1) nach einem der vorhergehenden Ansprüche, **dadurch gekennzeichnet, dass** jedes Saugloch (7, 9) eine zu dem Anschluss (5) hin stetig abnehmende Fläche seines freien Querschnitts aufweist.

12. Saugerspitze (1) nach einem der vorhergehenden Ansprüche, **dadurch gekennzeichnet, dass** ein freier Gesamtquerschnitt des Hauptkörpers (12) stetig in den freien Anschlussquerschnitt (6) der Saugerspitze (1) übergeht.

13. Saugerspitze (1) nach einem der vorhergehenden Ansprüche, **dadurch gekennzeichnet, dass** in einem Hohlraum (17) innerhalb des Hauptkörpers (12) ein offenporiger Schwamm (18) angeordnet ist, wobei die Sauglöcher (7, 9) durch den offenporigen Schwamm (18) hindurch mit den Anschluss (5) kommunizieren.

## Claims

1. Suction tip (1) for suctioning a biological fluid, the suction tip comprising
- a main body (12) having a main axis (3) and a wall (4),
- a connector (5) for a suction line (2), which is formed at the main body (12) on the main axis (3) and which defines a free connection cross section (6) of the suction tip (1), and
- a plurality of decentral suction holes (4) which laterally enter into the main body (12), which extend through the wall (4) of the main body (12) and which communicate with the connector (5) within the main body (12),
- wherein the decentral suction holes (7) laterally enter into the main body (12) at an acute angle of not more than 60° with respect to the main axis (3) in such a way that they are not directly positioned opposite to the connector (5) for the suction line (2) formed centrally on the main axis (3),
- wherein a free minimum cross section (10) of each decentral suction hole (7), in a continuous continuation of its course (13) through the wall (4) of the main body (12), completely overlaps with the free connection cross section (6) of the suction tip (1), and
- wherein a sum of areas of the free minimum cross sections (10) of the decentral suction holes (7) is at least as large as an area of the free connection cross section (6) of the suction tip (1),
**characterised in**
- **that** the main body (12) further comprises a central suction hole (9) which extends through the wall (4) along the main axis (3).

2. Suction tip (1) of claim 1, **characterised in that** a free minimum cross section (15) of the central suction hole (9) is equal to the free connection cross section (6) of the suction tip (1).

3. Suction tip (1) of any of the preceding claims, **characterised in that** the decentral suction holes (7) enter into the main body (12) on at least one virtual ring (16) around the main axis (3).

4. Suction tip (1) of claim 3, **characterised in that** a sum of areas of the free minimum cross sections (10) of the decentral suction holes (7) on each of the virtual rings (16) around the main axis (3) is at least as large as an area of the free connection cross section (6) of the suction tip (1).

5. Suction tip (1) of any of the preceding claims, **characterised in that** the free minimum cross sections (10) of the decentral suction holes (7), in the continuous continuations of their courses (13) through the wall (4) of the main body (12), are uniformly distributed over the free connection cross section (6) of the suction tip (1).

6. Suction tip (1) of any of the preceding claims **characterised in that** the free minimum cross sections (6) of the decentral suction holes (7) are circular.

7. Suction tip (1) of any of the preceding claims, **characterised in that** the decentral suction holes (7) enter into the main body (12) at an acute angle of not more than 45° with respect to the main axis (3).

8. Suction tip (1) of claim 7, **characterised in that** at least a plurality of the decentral suction holes (7) have a straight course (13) through the wall (4) of the main body (12).

9. Suction tip (1) of any of the preceding claims, **characterised in that** at least a plurality of the decentral suction holes (7) have a curved course (13) through the wall (4) of the main body (12).

10. Suction tip (1) of claim 9, **characterised in that** a radius of curvature of the curved course, related to a hole axis of the respective suction hole, is at least as long as a maximum diameter of the main body (12) perpendicular to its main axis (3).

11. Suction tip (1) of any of the preceding claims, **characterised in that** each suction hole (7, 9) has an area of its free cross section which steadily decreases towards the connector (5).

12. Suction tip (1) of any of the preceding claims, **characterised in that** a free total cross section of the main body (12) steadily passes into the free connection cross section (6) of the suction tip (1).

13. Suction tip (1) of any of the preceding claims, **characterised in that** an open-cell sponge (18) is arranged a cavity (17) with the main body (12), wherein the suction holes (7, 9) communicate with the connector (5) through the open-cell sponge (18).

## Revendications

1. Pointe de dispositif de succion (1) conçue pour aspirer un liquide biologique, avec
- un corps principal (12) comportant un axe principal (3) et une paroi (4),
- un raccord (5) pour une conduite d'aspiration (2), constitué sur l'axe principal (3) sur le corps principal (12) et définissant une section transversale de raccordement (6) libre de la pointe de dispositif de succion (1) et
- plusieurs trous d'aspiration (7) excentrés qui entrent latéralement dans le corps principal (12), s'étendent à travers la paroi (4) du corps principal (12) et communiquent dans le corps principal (12) avec le raccord (5),
- les trous d'aspiration (7) excentrés pénétrant latéralement dans le corps principal (12) en formant avec l'axe principal (3) un angle aigu ne dépassant pas 60° de telle sorte qu'ils ne sont pas directement en face du raccord (5) pour la conduite d'aspiration (2) qui est constitué de façon centrée sur l'axe principal (3),
- une section transversale minimale (10) libre de chaque trou d'aspiration (7) excentré chevauchant complètement la section transversale de raccordement (6) libre de la pointe de dispositif de succion (1) dans la continuité de son tracé (13) à travers la paroi (4) du corps principal (12), et
- une somme de surfaces des sections transversales minimales (10) libres des trous d'aspiration (7) excentrés étant au moins égale à une surface de la section transversale de raccordement (6) libre de la pointe de dispositif de succion (1),
**caractérisée en ce que**
- le corps principal (12) comporte en outre un trou d'aspiration (9) centré qui s'étend le long de l'axe principal (3) à travers la paroi (4).

2. Pointe de dispositif de succion (1) selon la revendication 1, **caractérisée en ce qu'**une section transversale minimale (15) libre du trou d'aspiration (9) centré coïncide avec la section transversale de raccordement (6) libre de la pointe de dispositif de succion (1).

3. Pointe de dispositif de succion (1) selon l'une des revendications précédentes, **caractérisée en ce que** les trous d'aspiration (7) excentrés pénètrent dans le corps principal (12) sur au moins un anneau (16) imaginaire autour de l'axe principal (3).

4. Pointe de dispositif de succion (1) selon la revendication 3, **caractérisée en ce qu'**une somme de surfaces des sections transversales minimales (10) libres des trous d'aspiration (7) excentrés sur chacun des anneaux (16) imaginaires autour de l'axe principal (3) est au moins égale à une surface de la section transversale de raccordement (6) libre de la pointe de dispositif de succion (1).

5. Pointe de dispositif de succion (1) selon l'une des revendications précédentes, **caractérisée en ce que** les sections transversales minimales (10) libres des trous d'aspiration (7) excentrés dans la continuité de leurs tracés (13) à travers la paroi (4) du corps principal (12) sont réparties de façon égale sur la section transversale de raccordement (6) libre de la pointe de dispositif de succion (1).

6. Pointe de dispositif de succion (1) selon l'une des revendications précédentes, **caractérisée en ce que** les sections transversales minimales (6) libres des trous d'aspiration (7) excentrés sont circulaires.

7. Pointe de dispositif de succion (1) selon l'une des revendications précédentes, **caractérisée en ce que** les trous d'aspiration (7) excentrés pénètrent dans le corps principal (12) en formant avec l'axe principal (3) un angle aigu ne dépassant pas 45°.

8. Pointe de dispositif de succion (1) selon la revendication 7, **caractérisée en ce qu'**au moins plusieurs des trous d'aspiration (7) excentrés présentent un parcours (13) rectiligne à travers la paroi (4) du corps principal (12).

9. Pointe de dispositif de succion (1) selon l'une des revendications précédentes, **caractérisée en ce qu'**au moins plusieurs des trous d'aspiration (7) excentrés présentent un parcours (13) courbe à travers la paroi (4) du corps principal (12).

10. Pointe de dispositif de succion (1) selon la revendication 9, **caractérisée en ce qu'**un rayon de courbure du parcours courbe par rapport à un axe de trou du trou respectif est au moins égal à un diamètre maximal du corps principal (12) perpendiculairement à son axe principal (3).

11. Pointe de dispositif de succion (1) selon l'une des revendications précédentes, **caractérisée en ce que** chaque trou d'aspiration (7, 9) comporte une surface de sa section transversale libre qui diminue constamment en direction du raccord (5).

12. Pointe de dispositif de succion (1) selon l'une des revendications précédentes, **caractérisée en ce qu'**une section transversale totale libre du corps principal (12) se transforme de façon continue en section transversale de raccordement (6) libre de la pointe de dispositif de succion (1).

13. Pointe de dispositif de succion (1) selon l'une des revendications précédentes, **caractérisée en ce qu'**une éponge (18) à pores ouverts est disposée dans une cavité (17) à l'intérieur du corps principal (12), les trous d'aspiration (7, 9) communiquant avec le raccord (5) à travers l'éponge (18) à pores ouverts.
